(19) Europäisches Patentamt
European Patent Office
Office européen des brevets

(11) Publication number: **0 324 543 A2**

# (12) EUROPEAN PATENT APPLICATION

(21) Application number: **89300082.8**

(22) Date of filing: **05.01.89**

(51) Int. Cl.[4]: **C07D 487/08 , A61K 31/40 , //(C07D487/08,209:00,209:00)**

Claims for the following Contracting States: ES + GR.

(30) Priority: **13.01.88 GB 8800694**

(43) Date of publication of application: **19.07.89 Bulletin 89/29**

(84) Designated Contracting States: **AT BE CH DE ES FR GB GR IT LI LU NL SE**

(71) Applicant: **Pfizer Limited**
**Ramsgate Road**
**Sandwich Kent CT13 9NJ(GB)**

(72) Inventor: **Arrowsmith, John Edmund, Dr.**
**340 Dover Road Upper Walmer**
**Deal Kent(GB)**
Inventor: **Campbell, Simon Fraser, Dr.**
**Church House Churchcliffe Road**
**Kingsdown Deal Kent(GB)**
Inventor: **Cross, Peter Edward, Dr.**
**21, Cherry Avenue**
**Canterbury Kent(GB)**
Inventor: **Dickinson, Roger Peter**
**6, Kingston Close**
**River Dover Kent(GB)**
Inventor: **MacKenzie, Alexander Roderick, Dr.**
**48, St. Leonards Road**
**Deal Kent(GB)**

(74) Representative: **Wood, David John et al**
**Pfizer Limited Ramsgate Road**
**Sandwich Kent CT13 9NJ(GB)**

## (54) Antiarrhythmic agents.

(57) The invention provides antiarrhythmic agents of the formula:-

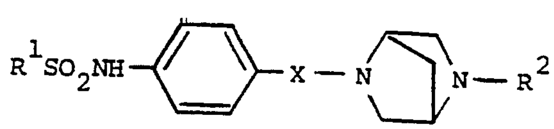

--- (I)

or a pharmaceutically acceptable salt thereof, wherein $R^1$ is $C_1$-$C_4$ alkyl; X is $-(CH_2)_2-$, $-CH(OH)CH_2-$, $-COCH_2-$ or a direct link; and $R^2$ is 4-pyridyl or 4-amino-2-pyridyl.

EP 0 324 543 A2

## ANTIARRHYTHMIC AGENTS

This invention relates to certain 2,5-diazabicyclo[2.2.1]heptanes which are antiarrhythmic agents, and to intermediates therefor.

The antiarrhythmic agents of the invention prolong the duration of the action potential in cardiac muscle and conducting tissue, and thereby increase refractoriness to premature stimuli. Thus, they are Class III antiarrhythmic agents according to the classification of Vaughan Williams (Anti-Arrhythmic Action, E.M. Vaughan Williams, Academic Press, 1980). They are effective in atria, ventricles and conducting tissue both in vitro and in vivo and are therefore useful for the prevention and treatment of a wide variety of ventricular and supraventricular arrhythmias including atrial and ventricular fibrillation. Because they do not alter the speed at which impulses are conducted, they have less propensity than current drugs (mostly Class I) to precipitate or aggravate arrhythmias, and also produce less neurological side effects. Some of the compounds also have some positive inotropic activity and therefore are particularly beneficial in patients with impaired cardiac pump function.

Thus the invention provides antiarrhythmic agents of the formula:-

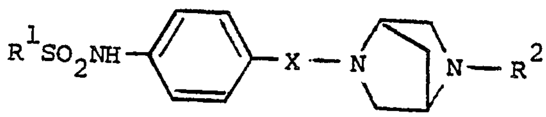

--- (I)

and their pharmaceutically acceptable salts,
where $R^1$ is $C_1$-$C_4$ alkyl;
X is $-(CH_2)_2-$, $-CH(OH)CH_2-$, $-COCH_2-$ or a direct link,
and $R_2$ is a group of the formula:-

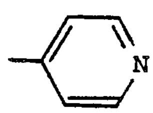

or

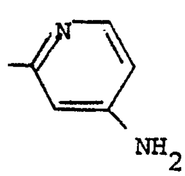

$R^1$ is preferably methyl. $R^2$ is preferably 4-pyridyl. X is preferably $-CH(OH)CH_2-$ or a direct link.

The pharmaceutically acceptable salts of the compounds of the formula (I) include acid addition salts formed from acids which form non-toxic acid addition salts containing pharmaceutically acceptable anions, such as hydrochloride, hydrobromide, hydroiodide, sulphate or bisulphate, phosphate or hydrogen phosphate, acetate, maleate, fumarate, lactate, tartrate, citrate, gluconate, benzoate, methanesulphonate, besylate and p-toluenesulphonate salts. Also included are the alkali metal salts, particularly the sodium and potassium salts. The salts are preparable by conventional techniques.

For assessment of effects of the compounds on atrial refractoriness, guinea pig right hemiatria are mounted in a bath containing physiological salt solution, and one end is connected to a force transducer. Tissues are stimulated at 1 Hz using field electrodes. Effective refractory period (ERP) is measured by introducing premature stimuli ($S_2$) after every 8th basic stimulus ($S_1$). The $S_1S_2$ coupling interval is gradually increased until $S_2$ reproducibly elicits a propagated response. This is defined as the ERP. The concentration of compound required to increase ERP by 25% ($ED_{25}$) is then determined. ERP is also measured in guinea pig right papillary muscles incubated in physiological salt solution. Muscles are stimulated at one end using bipolar electrodes and the propagated electrogram is recorded at the opposite end via a unipolar surface electrode. ERP is determined as above using the extrastimulus technique. Conduction time is obtained from a digital storage oscilloscope by measuring the interval between the stimulus artefact and the peak of the electrogram (i.e. the time required for the impulse to travel along the length of the muscle).

Atrial and ventricular ERP's are also measured in anaesthetised or conscious dogs by the extrastimulus technique whilst the atrium or right ventricle is being paced at a constant rate.

The compounds of the formula (I) can be administered alone but will generally be administered in admixture with a pharmaceutical carrier selected with regard to the intended route of administration and standard pharmaceutical practice. They can be administered both to patients suffering from arrhythmias and

also prophylactically to those likely to develop arrhythmias. For example they may be administered orally in the form of tablets containing such excipients as starch of lactose, or in capsules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other solutes, for example, enough salts or glucose to make the solution isotonic.

For administration to man in the curative or prophylactic treatment of cardiac conditions such as ventricular and supraventricular arrhythmias, including atrial and ventricular fibrillation, it is expected that oral dosages of the compounds of the formula (I) will be in the range from 2 to 150 mg daily, taken in and up to 4 divided doses per day, for an average adult patient (70 kg). Dosages for intravenous administration would be expected to be within the range 1.0 to 20mg per single dose as required. A severe cardiac arrythmia is preferably treated by the i.v. route in order to effect a rapid conversion to the normal rhythm. Thus for a typical adult patient individual tablets or capsules might contain 2 to 50mg of active compound, in a suitable pharmaceutically acceptable vehicle or carrier. Variations may occur depending on the weight and condition of the subject being treated as will be known to medical practitioners.

Thus the present invention provides a pharmaceutical composition comprising a compound of the formula (I) as defined above or pharmaceutically acceptable salt thereof, together with a pharmaceutically acceptable diluent or carrier.

The invention yet further provides a compound of the formula (I) or a pharmaceutically acceptable salt thereof, for use as a medicament.

Where the compounds of the formula (I) contain one or more asymmetric centres, then the invention includes both the unseparated and separated forms, including diastereomers.

The invention also provides the use of a compound of the formula (I), or of a pharmaceutically acceptable salt thereof, for the manufacture of a medicament for the prevention or reduction of cardiac arrhythmias.

The compounds of the formula (I) in which $R^2$ is 4-pyridyl and $R^1$ and X are as defined for formula (I) can be prepared by the acylation of compounds of the formula:-

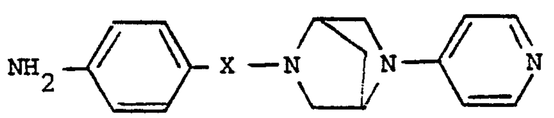

--- (II)

where X is as defined for formula (I),
using a $C_1$-$C_4$ alkanesulfonyl chloride or bromide or a $C_1$-$C_4$ alkanesulphonic anhydride in a suitable organic solvent and preferably in the presence of an acid acceptor such as pyridine, triethylamine, potassium carbonate or sodium bicarbonate. It is preferred to carry out the reaction using a $C_1$-$C_4$ alkanesulphonyl chloride in pyridine as the latter functions both as the acid acceptor and as the solvent. The reaction is typically carried out at about room temperature. The product of the formula (I) can then be isolated and purified by conventional techniques.

The starting materials of the formula (II), which also form a part of this invention, can be prepared by conventional techniques, some of which are illustrated in the following Preparations.

The compounds of the formula (I) in which $R^1$ is $C_1$-$C_4$ alkyl, $R^2$ is 4-pyridyl and X is as defined for formula (I) can also be prepared as follows:-

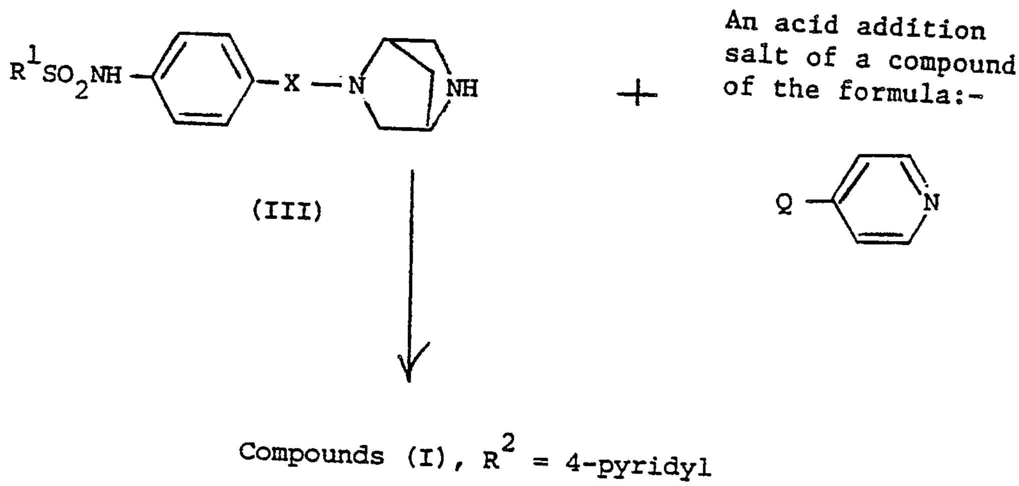


(III)

Q is a leaving group such as chloro, bromo, iodo, methanesulphonyloxy, benzenesulphonyloxy or toluenesulphonyloxy. It is strongly preferred to carry out the reaction using the pyridine derivative as an addition salt to prevent self-polymerisation. (It is in fact preferred to use 4-chloropyridine hydrochloride.)

The reaction is typically carried out with heating at, say, 60-130 °C and in the presence of an acid acceptor such as triethylamine. Water is a suitable solvent. The product can then be isolated and purified conventionally.

The starting materials (III), which form a part of the invention, can again be prepared by routine methods as will be known to those skilled in the art, some of these being illustrated in the following Preparations.

The compounds of the formula (I) in which $R^2$ is 4-amino-2-pyridyl can be prepared by the reduction of the corresponding 4-nitro-2-pyridyl compounds using e.g. $H_2/Pd/C$. The 4-nitro-2-pyridyl starting materials can also be prepared conventionally, e.g. as follows:-

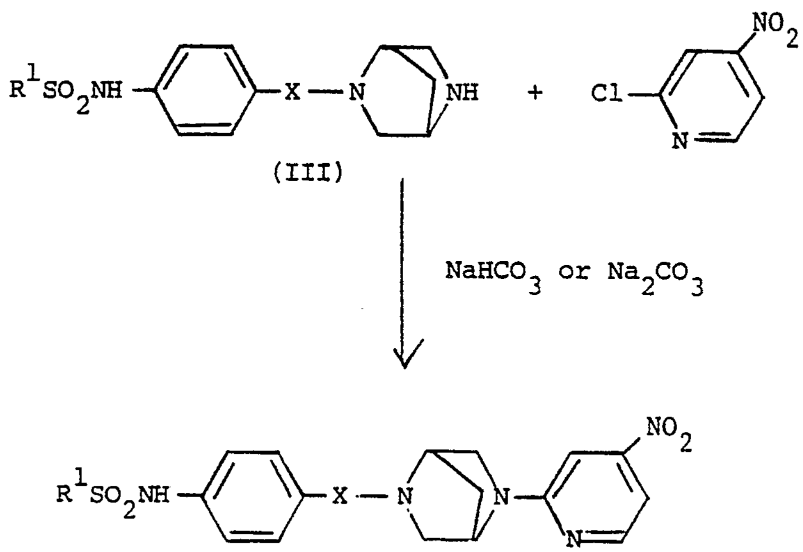


(III)

Replacement of the 2,5-diazabicyclo[2.2.1]heptane ring with one of the following also leads to antiarrhythmic agents which can again be prepared routinely:-

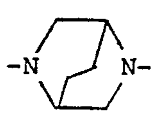 , 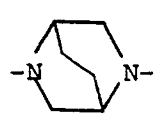 , 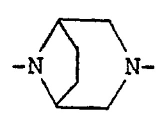 and 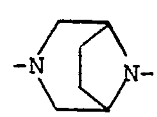 .

The following Examples, in which all temperatures are in °C, illustrate the invention:-

## EXAMPLE 1

Preparation of 2-[2-hydroxy-2-(4-methanesulphonamidophenyl)ethyl]5-(4-pyridyl)-2,5-diazabicyclo[2.2.1]-heptane

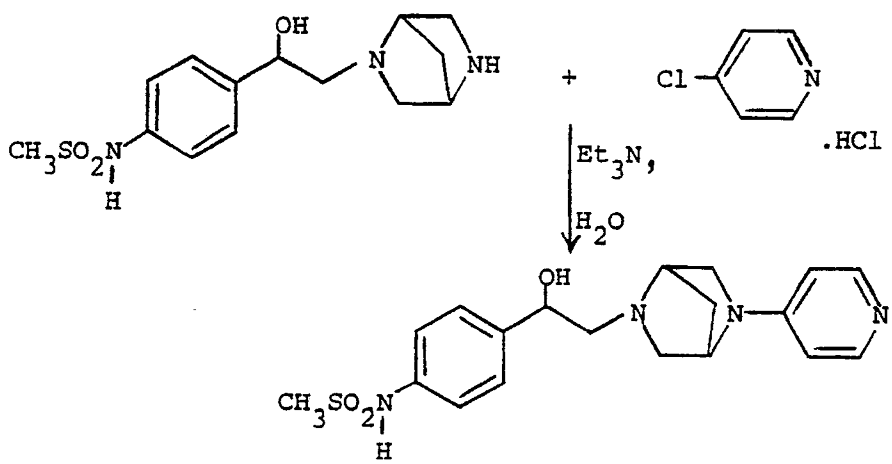


A stirred solution of 2-[2-hydroxy-2-(4-methanesulphonamidophenyl)ethyl]-2,5-diazabicyclo[2.2.1]-heptane (0.18 g), 4-chloropyridine hydrochloride (0.086 g) and triethylamine (0.12 g) in water (2 ml) was heated at 120° under a nitrogen atmosphere in a bomb for 5 hours. After cooling, the solvent was removed in vacuo and the residue purified by column chromatography on silica eluting with methylene chloride containing methanol (10% to 15%). The product-containing fractions were combined and evaporated to give an oil which crystallised to give the title compound as a mixture of diastereomers, yield 0.019, m.p. 125-130°.

$^1$H N.m.r. ($CD_3OD$), $\delta$ = 8.10 (d, 2H); 7.30 (q, 4H); 6.7 (d, 2H); 4.70 (m, 1H); 3.45 (m, 3H); 3.10 (q, 2H); 2.95 (s, 3H); 2.85 (m, 3H); 2.65 (d, 1H); 2.10 (t, 1H); 1.12 (t, 1H).

Mass spectrum, m/e = 388.

## EXAMPLE 2

Preparation of 2-(4-methanesulphonamidophenyl)-5-(4-pyridyl)-2,5-diazabicyclo[2.2.1]heptane

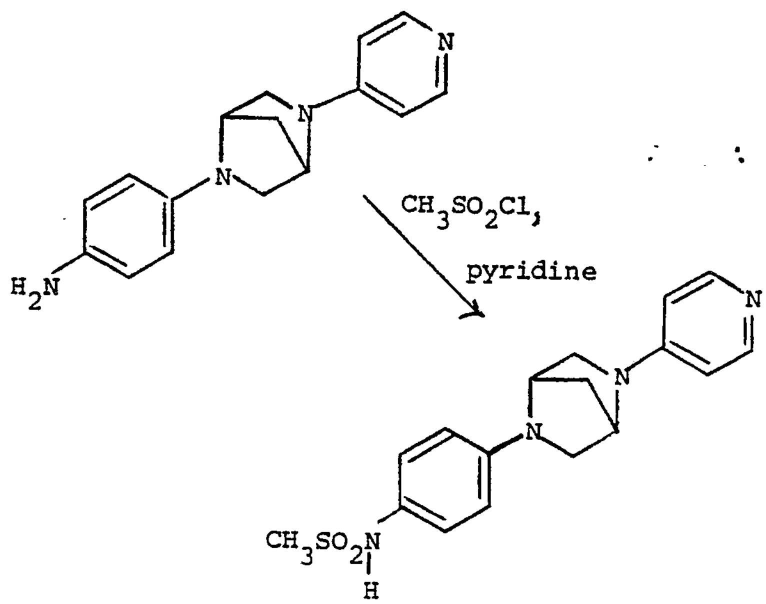


Methanesulphonyl chloride (0.1 ml) was added to a stirred mixture of 2-(4-aminophenyl)-5-(4-pyridyl)-2,5-diazabicyclo[2.2.1]heptane (0.3 g) in dry pyridine (5 ml) at 0°. The solution was allowed to warm to room temperature and stirred for 3 days. The mixture was concentrated in vacuo and the residue partitioned between saturated aqueous sodium bicarbonate and dichloromethane. The layers were separated and the aqueous layer extracted three times with dichloromethane. The dichloromethane extracts were combined, dried ($MgSO_4$) and concentrated in vacuo. The resulting oil was purified by column chromatography on silica gel eluting with dichloromethane containing methanol (0% up to 5%). The product-containing fractions were combined and concentrated in vacuo. The resulting solid was crystallised from ethyl acetate to give the title compound, yield 0.07 g.

$^1$H N.m.r ($CDCl_3$) $\delta$ = 8.25 (d, 2H); 7.15 (d, 2H); 6.55 (d, 2H); 6.40 (d, 2H; 4.55 (s, 1H); 4.50 (s, 1H); 3.70 (d, 1H); 3.60 (d, 1H); 3.35 (d, 1H); 3.20 (d, 1H); 2.95 (s, 3H); 2.20 (ABq, 2H) ppm.

The following Preparations, in which all temperatures are in °C, illustrate the preparation of the novel starting materials.

## Preparation 1

Preparation of trans-1-benzyloxycarbonyl-4-hydroxy-2-hydroxymethylpyrrolidine [see also Heterocycles, 22, 2735 (1984)]

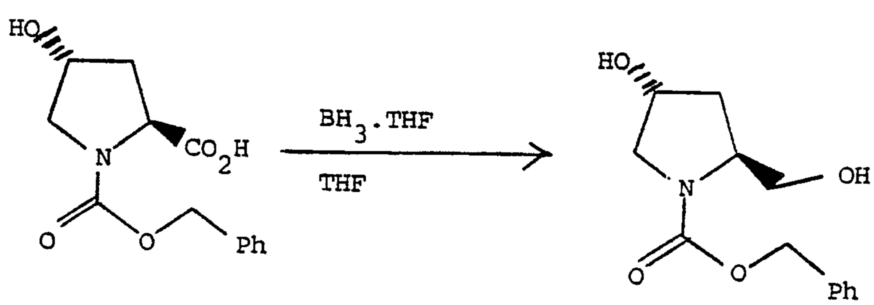


To a solution of borane-tetrahydrofuran complex (270 ml of a 1.0 molar solution in tetrahydrofuran) at 0°, was added, dropwise over 40 minutes, a solution of trans-1-benzyloxycarbonyl-4-hydroxy-2-pyrrolidinecarboxylic acid (commercially available) (35 g) in tetrahydrofuran (200 ml). When the addition was

complete, the mixture was allowed to warm to room temperature and then heated under reflux for 3 hours. The mixture was cooled to 0° and a further quantity of borane-tetrahydrofuran complex (270 ml of a 1.0 molar solution in tetrahydrofuran) was added, dropwise, over 10 minutes. The solution was heated under reflux for 3.5 hours and then allowed to cool to room temperature. The reaction was quenched by the addition of 6M hydrochloric acid (70 ml) and the mixture concentrated in vacuo. The residue was dissolved in water (500 ml) and extracted with dichloromethane (3 x 200 ml). The dichloromethane extracts were combined, washed with 10% aqueous sodium carbonate solution, dried ($MgSO_4$) and concentrated in vacuo to give the title compound as a gum, yield 27g.

$^1$H N.m.r. ($CDCl_3$) $\delta$ = 7.45-7.25 (m, 5H); 5.15 (s, 2H); 4.85 (m, 1H); 4.40 (m, 1H); 4.20 (m, 1H); 3.80-3.40 (m, 4H); 3.00 (m, 1H), 2.10-2.00 (m, 1H), 1.80-1.65 (m, 1H) ppm.

## Preparation 2

### Preparation of trans-1-benzyloxycarbonyl-4-(p-tosyloxy)-2-(p-tosyloxymethyl)pyrrolidine

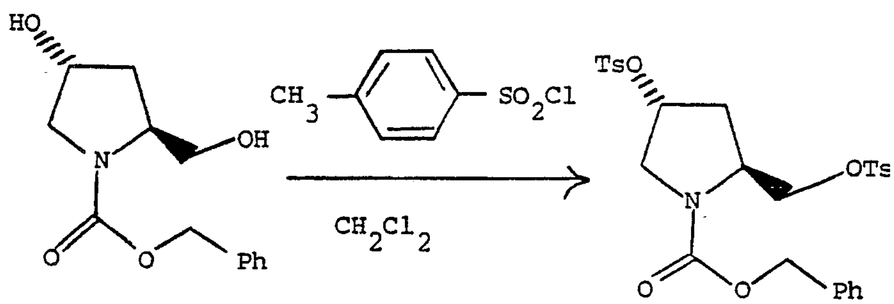


Para-toluenesulphonyl chloride (50 g) was dissolved in dry dichloromethane (100 ml). Pyridine (24 ml) was added and the mixture cooled to 0°. A solution of trans-1-benzyloxycarbonyl-4-hydroxy-2-hydroxymethylpyrrolidine (27 g) in dichloromethane (100 ml) was added and the mixture stirred at 0° for 2 hours then stored at 4° for 3 days. The solution was concentrated in vacuo to approximately half the original volume, pyridine (20 ml) was added and the mixture stirred at 0° for 6 hours then warmed to room temperature and stirred for 24 hours. The mixture was concentrated in vacuo and the residue stirred with ice/water for 15 minutes. Water (300 ml) and concentrated hydrochloric acid (100 ml) were added and the mixture was extracted twice with dichloromethane. The dichloromethane extracts were combined, dried ($MgSO_4$) and concentrated in vacuo to leave the title compound as a gum, yield 60 g.

$^1$H N.m.r. ($CDCl_3$) $\delta$ = 7.80-7.65 (m, 4H); 7.40-7.20 (m, 9H); 5.10-4.95 (ABq, 2H); 4.45 (dd, 1H); 4.20 (m, 2H); 4.05 (d, 1H); 3.75 (d, 1H); 3.50 (dd, 1H); 2.47 (s, 3H); 2.42 (s, 3H); 2.20 (m, 2H); ppm.

## Preparation 3

### Preparation of 2-benzyl-5-benzyloxycarbonyl-2,5-diazabicyclo[2.2.1]heptane

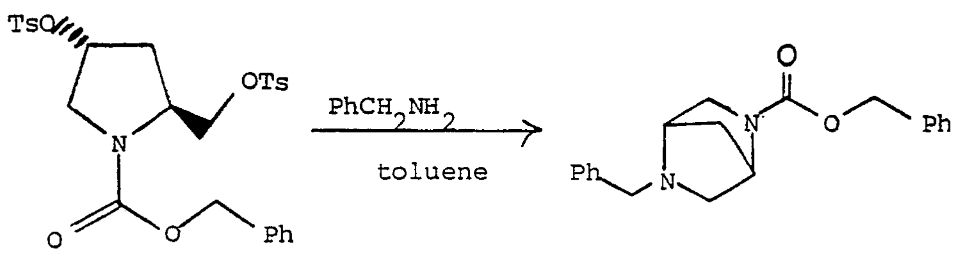

A solution of trans-1-benzyloxycarbonyl-4-(p-tosyloxy)-2-(p-tosyloxymethyl)-pyrrolidine (60 g) and benzylamine (35 g) in toluene (500 ml) was heated under reflux for 14 hours then allowed to stand at room temperature for 72 hours. The solid was filtered off and washed with toluene. The filtrate and washings were combined and concentrated in vacuo. The resulting oil was purified by column chromatography on silica gel eluting with dichloromethane containing methanol (0% up to 5%). The product-containing fractions were combined and concentrated in vacuo to give the title compound as an oil, yield 22 g.
$^1$H N.m.r. ($CDCl_3$) $\delta$ = 7.50-7.20 (m, 10H); 5.20 (s, 2H); 4.50-4.40 (m, 1H); 3.85-3.60 (m, 3H); 3.55 (d, 1H); 3.25 (d, 1H); 2.95 (m, 1H); 2.80-2.60 (m, 1H); 1.95 (d, 1H); 1.75 (t, 1H), ppm.

## Preparation 4

### Preparation of 2-benzyl-2,5-diazabicyclo[2.2.1]heptane

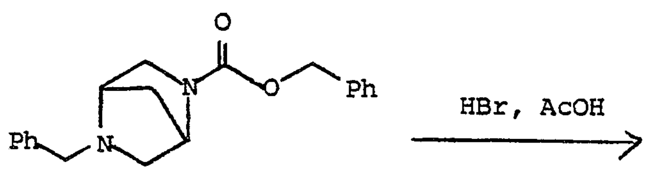


A solution of 2-benzyl-5-benzyloxycarbonyl-2,5-diazabicyclo[2.2.1]heptane (5 g) in 45% hydrobromic acid in acetic acid (60 ml) was heated at 50-60° for l7 hours. On cooling to room temperature, the mixture was diluted with dry ether (450 ml). The resulting grey solid was filtered off and dried in vacuo. The solid was ground together with sodium hydroxide pellets (7 g) under ether. Methanol (5 ml) was added and the mixture sonicated for 1 hour then filtered and the solid washed with ether. The filtrate and washings were combined and concentrated in vacuo to give the title compound as an oil, yield 2.5 g.
$^1$H N.m.r. ($CDCl_3$) $\delta$= 7.40-7.20 (m, 5H); 3.75 (ABq, 2H); 3.55 (m, 1H); 3.40 (s, 1H); 3.25 (dd, 1H); 3.00-2.80 (m, 2H); 2.45 (m, 1H); 1.85 (m, 1H); 1.60 (m, 2H) ppm.

## Preparation 5

### Preparation of 2-benzyl-5-(4-pyridyl)-2,5-diazabicyclo[2.2.1]heptane

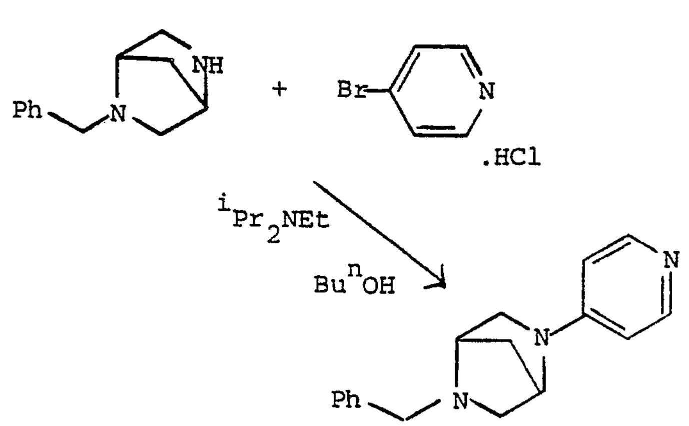


A solution containing 2-benzyl-2,5-diazabicyclo[2.2.1]heptane (3.7 g), 4-bromopyridine hydrochloride (3.83 g) and N,N-diisopropylethylamine (15 ml) in n-butanol (15 ml) was heated under reflux for 2 days. On cooling, the mixture was concentrated in vacuo and the residue partitioned between dichloromethane and

10% aqueous sodium carbonate. The layers were separated and the dichloromethane layer was dried ($MgSO_4$) and concentrated in vacuo. The resulting oil was purified by column chromatography on silica gel eluting with toluene containing ethanol (0% up to 10%). The product-containing fractions were combined and concentrated in vacuo to give the title compound as a buff solid, yield 2.2 g.

$^1$H N.m.r. ($CDCl_3$) $\delta$ = 8.20 (d, 2H); 7.40-7.20 (m, 5H); 6.40 (d, 2H); 4.30 (s, 1H); 3.75 (s, 2H); 3.60 (s, 1H); 3.45-3.30 (m, 2H); 2.95 (d, 1H); 2.65 (d, 1H); 2.05 (d, 1H); 1.85 (d, 1H) ppm.

## Preparation 6

### Preparation of 2-(4-pyridyl)-2,5-diazabicyclo[2.2.1]heptane

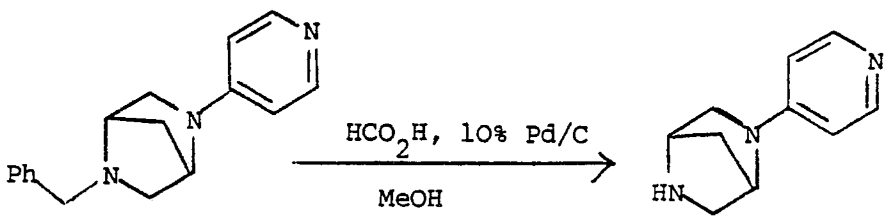


To a solution of 2-benzyl-5-(4-pyridyl)-2,5-diazabicyclo[2.2.1]heptane (2.2 g) in 5% formic acid in methanol (100 ml) at 0°, was added, in portions over 5 minutes, 10% palladium on carbon (2 g). The mixture was allowed to warm to room temperature and stirred for 5 days. Dichloromethane (100 ml) was added and the mixture filtered. The filtrate was concentrated in vacuo to give the title compound, yield 1.3 g.

$^1$H N.m.r. ($CDCl_3$) $\delta$ = 8.20 (d, 2H); 6.40 (d, 2H); 4.40 (s, 1H); 3.90 (s, 1H); 3.60 (d, 1H); 3.10 (m, 2H); 2.15 (brs, 2H); 1.9 (ABq, 2H) ppm.

## Preparation 7

### Preparation of 2-(4-nitrophenyl)-5-(4-pyridyl)-2,5-diazabicyclo[2.2.1]heptane

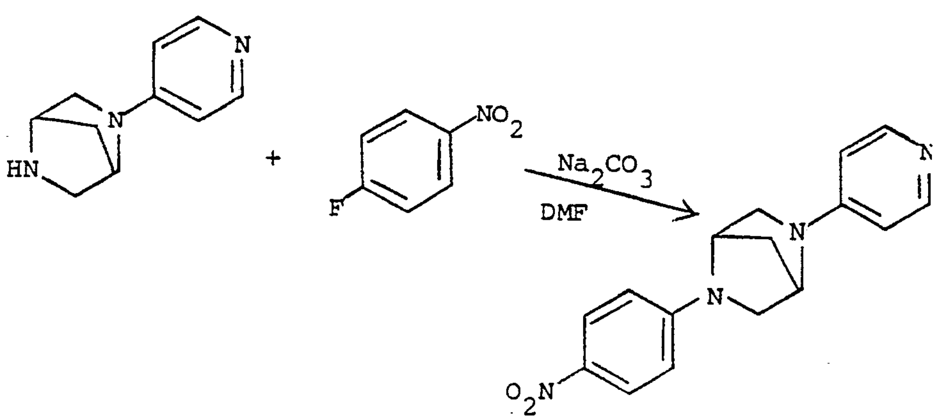


A mixture containing 2-(4-pyridyl)-2,5-diazabicyclo[2.2.1]heptane (1.3 g), 4-fluoronitrobenzene (1.05 g), powdered anhydrous sodium carbonate (0.8 g) and dimethylformamide (10 ml) was heated at 100° for 1.5 hours. The mixture was concentrated in vacuo and the residue partitioned between water and dichloro methane. The layers were separated and the aqueous layer extracted four times with 5% ethanol in dichloromethane. The organic extracts were combined, dried ($MgSO_4$) and concentrated in vacuo. The

resulting gum was purified by column chromatography on silica gel eluting with dichloromethane containing methanol (0% up to 10%). The product-containing fractions were combined and concentrated in vacuo to give the title compound as a gum, yield 0.75 g.

$^1$H N.m.r ($CDCl_3$) δ= 8.25 (d, 2H); 8.15 (d, 2H); 6.50 (d, 2H); 6.45 (d, 2H); 4.75 (s, 1H); 4.65 (s, 1H); 3.80-3.60 (m, 2H); 3.40-3.30 (m, 2H); 2.25 (s, 2H) ppm.

Preparation 8

Preparation of 2-(4-aminophenyl)-5-(4-pyridyl)-2,5-diazabicyclo[2.2.1]heptane

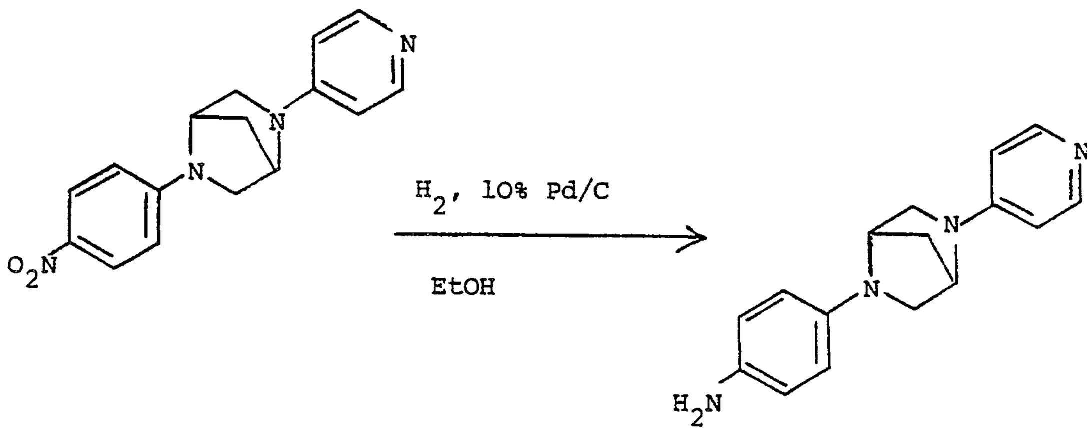


A mixture of 2-(4-nitrophenyl)-5-(4-pyridyl)-2,5-diazabicyclo[2.2.1]heptane (0.75 g) and 10% palladium-on-carbon (0.075 g) in ethanol (50 ml) was hydrogenated at room tempeature and 30 p.s.i. (206.8 kPa) pressure for 3 hours. A further 0.05 g of 10% palladium-on-carbon was added and hydrogenation was continued for 6 hours. The mixture was then diluted with dichloromethane and filtered. The filtrate was concentrated in vacuo and the residual oil purified by column chromatography on silica gel, eluting with dichloromethane containing methanol (0% up to 5%). The product-containing fractions were combined and concentrated in vacuo. The resulting solid was crystallised from ethanol to give the title compound, yield 0.45 g, m.p. 236-242°.

$^1$H N.m.r. ($CDCl_3$) δ = 8.20 (d, 2H); 6.65 (d, 2H); 6.45 (d, 2H); 6.35 (d, 2H); 4.50 (s, 1H); 4.45 (s, 1H); 3.70 (d, 1H); 3.55 (d, 1H); 3.40-3.20 (m, 3H); 3.10 (d, 1H); 2.15 (ABq, 2H) ppm.

Preparation 9

Preparation of 2-benzyl-5-[4-methanesulphonamidobenzoylmethyl]-2,5-diazabicyclo[2.2.1]heptane

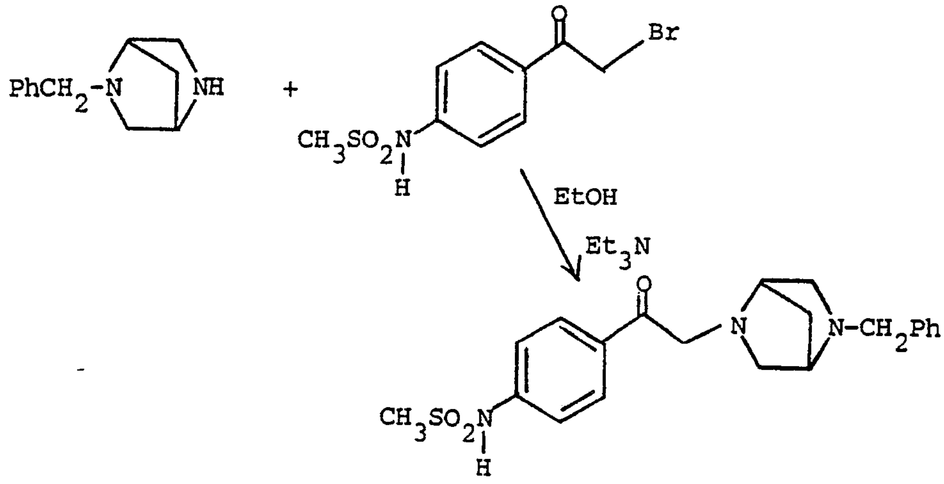


A mixture of 2-benzyl-2,5-diazabicyclo[2.2.1]heptane (2.65 g) (see Preparation 4), 4-methanesulphonamidophenacyl bromide (4.11 g) and triethylamine (2 ml) in ethanol (70 ml) was stirred at room temperature for 18 hours. The solvent was evaporated in vacuo and the residue taken up in methylene chloride, then washed (saturated aqueous sodium bicarbonate and brine), dried ($MgSO_4$) and evaporated to dryness in vacuo. The resulting oil was purified by column chromatography on silica eluting with methylene chloride containing methanol (0% up to 4%). The product-containing fractions were combined and evaporated in vacuo to give the title compound as a foam, yield 2.33 g.
$^1H$ N.m.r. ($CDCl_3$) $\delta$ = 8.0 (d, 2H); 7.35 (d, 2H); 7.30 (s, 5H); 4.10 (s, 2H); 3.80 (q, 2H); 3.55 (s, 1H); 3.40 (s, 1H); 3.05 (s, 3H); 3.00 (d, 2H); 2.90 (d, 1H); 2.70 (d, 1H); 1.85 (s, 2H) ppm.

## Preparation 10

Preparation of 2-benzyl-5-[2-hydroxy-2-(4-methanesulphonamidophenyl)ethyl]-2,5-diazabicyclo[2.2.1]-heptane hemihydrate

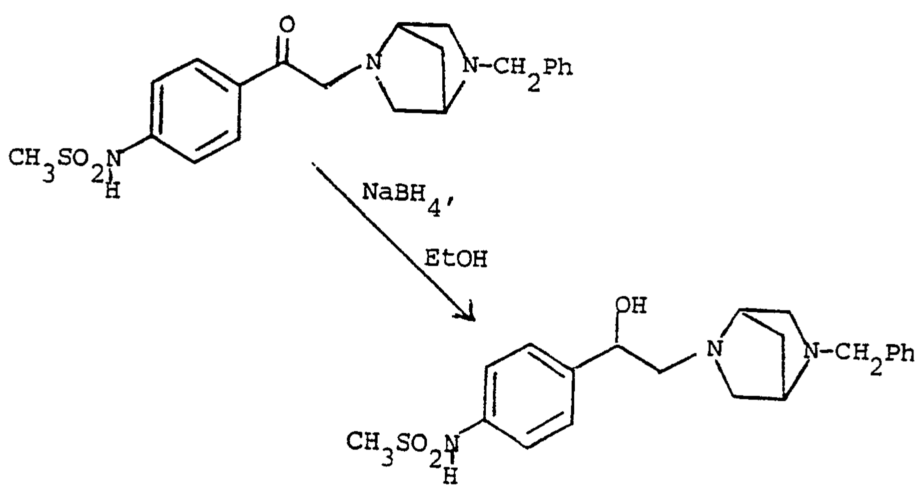


Sodium borohydride (1.2 g) was added to a stirred solution of 2-benzyl-[4-methanesulphonamidobenzoylmethyl]-2,5-diazabicyclo2.2.1]heptane (2.33 g) in ethanol (30 ml) at room temperature. Stirring was continued for 1½ hours and the reaction mixture was then evaporated in vacuo and the residue taken up in methylene chloride and 2M hydrochloric acid. The mixture was shaken and then saturated with sodium bicarbonate. The organic layer was separated and the aqueous layer was re-extracted with methylene chloride. The combined methylene chloride extracts were dried ($MgSO_4$) and

evaporated in vacuo to give a foam which was purified by column chromatography on silica eluting with methylene chloride containing methanol (2% up to 10%). The product-containing fractions were combined and evaporated in vacuo to give the title compound as a foam, yield 1.5 g.

| | | | |
|---|---|---|---|
| Found: | C,61.45; | H,6.7; | N,10.1; |
| Calculated for $C_{21}H_{27}N_3O_3S.\frac{1}{2}H_2O$: | C,61.4; | H,6.9; | N,10.2. |

$^1$H N.m.r. ($CDCl_3$) $\delta$ = 7.30 (m, 9H); 4.60 (d, 1H); 3.75 (q, 2H); 3.40 (s, 2H); 3.00 (s, 3H); 2.9 (m, 5H); 2.60 (dt, 1H); 1.80 (dq, 2H) ppm.

## Preparation 11

### Preparation of 2-[2-hydroxy-2-(4-methanesulphonamidophenyl)ethyl]-2,5-diazabicyclo[2.2.1]heptane

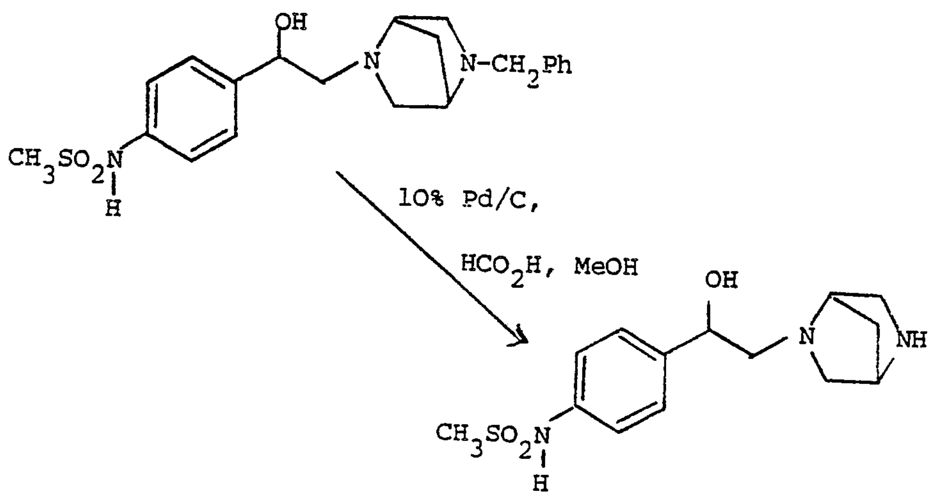


A solution of 2-benzyl-5-[2-hydroxy-2-(4-methanesulphonamidophenyl)ethyl]-2,5-diazabicyclo[2.2.1]-heptane (0.5 g) was stirred in 30 ml of 5% formic acid in methanol containing 10% Pd/C (0.3 g) for 1½ hours. The reaction mixture was filtered and the filt rate was evaporated in vacuo to give an oil which was purified by column chromatography on alumina eluting with methylene chloride containing methanol (10% up to 20%). The product-containing fractions were combined and evaporated in vacuo to give the title compound as an oil, yield 0.18 g.

$^1$H N.m.r. (DMSO $d_6$) $\delta$ = 7.20 (q, 4H); 4.45 (t, 1H); 3.4 (s, 2H); 3.35 (d, 1H); 2.95 (s, 3H); 2.8 (d, 1H); 2.50 (m, 5H); 1.60 (t, 1H); 1.40 (t, 1H).

## Claims

1. A compound of the formula:-

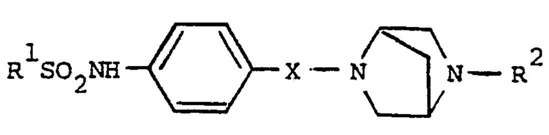
--- (I)

or a pharmaceutically acceptable salt thereof, wherein $R^1$ is $C_1$-$C_4$ alkyl; X is $-(CH_2)_2-$, $-CH(OH)CH_2-$, $-COCH_2-$ or a direct link; and $R^2$ is 4-pyridyl or 4-amino-2-pyridyl.

2. A compound of the formula (I) as claimed in claim 1, wherein $R^1$ is methyl.

3. A compound of the formula (I) as claimed in claim 1 or 2, wherein $R^2$ is 4-pyridyl.

4. A compound of the formula (I) as claimed in any one of claims 1 to 3, wherein X is $-CH(OH)CH_2-$ or a direct link.

5. 2-[2-Hydroxy-2-(4-methanesulphonamidophenyl)-ethyl]-5-(4-pyridyl)-2,5-diazabicyclo[2.2.1]heptane or a pharmaceutically acceptable salt thereof.

6. A compound of the formula:

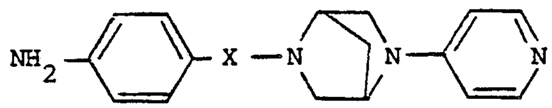

--- (II)

wherein X is as defined for formula (I).

7. A compound of the formula:

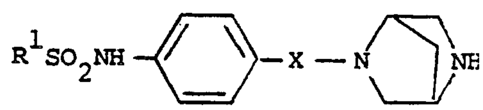

(III)

wherein $R^1$ and X are as defined for formula (I).

8. A pharmaceutical composition comprising a compound of the formula (I) as claimed in any one of claims 1 to 5, or a pharmaceutically acceptable salt thereof, together with a pharmaceutically acceptable diluent or carrier.

9. A compound of the formula (I), or a pharmaceutically acceptable salt or composition thereof, for use as a medicament.

10. The use of a compound of the formula (I), or a pharmaceutically acceptable salt or composition thereof, for the manufacture of a medicament for the prevention or reduction of cardiac arrhythmias.

Claims for the following Contracting State: ES

1. A process for preparing a compound of the formula:-

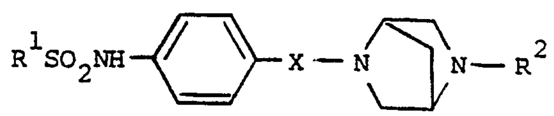

--- (I)

or a pharmaceutically acceptable salt thereof, wherein $R^1$ is $C_1$-$C_4$ alkyl; X is $-(CH_2)_2-$, $-CH(OH)CH_2-$, $-COCH_2-$ or a direct link; and $R^2$ is 4-pyridyl, comprising the reaction of a compound of the formula:-

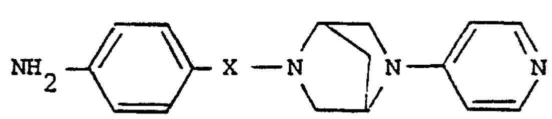

--- (II)

wherein X is as defined for formula (I), with either (a) a $C_1$-$C_4$ alkanesulphonyl chloride or bromide; or (b) a $C_1$-$C_4$ alkanesulphonic anhydride, followed by, optionally, conversion of the product into a pharmaceutically acceptable salt.

2. A process as claimed in claim 1 wherein a compound of the formula (II) is reacted with a $C_1$-$C_4$ alkanesulphonyl chloride in pyridine.

3. A process for preparing a compound of the formula (I) as defined in claim 1, or a pharmaceutically acceptable salt thereof, wherein $R^2$ is 4-pyridyl or 4-amino-2-pyridyl and $R^1$ and X are as defined for formula (I), comprising the reaction of a compound of the formula:

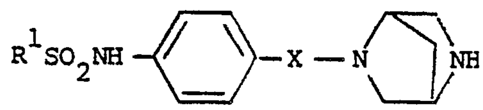

(III)

wherein $R^1$ and X are as defined for formula (I), with, as appropriate, either
(a) an acid addition salt of a compound of the formula:-

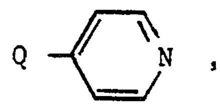

wherein Q is a suitable leaving group, in the presence of a suitable acid acceptor; or
(b) 2-chloro-4-nitropyridine in the presence of a suitable acid acceptor, followed by reduction of the resulting intermediate wherein $R^2$ 4-nitro-2-pyridyl to give a compound of the formula (I) in which $R^2$ is 4-amino-2-pyridyl;
said process (a) or (b) being followed by, optionally, conversion of the product into a pharmaceutically acceptable salt.

4. A process according to claim 3(a), wherein Q is Cl, the acid addition salt is the hydrochloride, and the acid acceptor triethylamine.

5. A process according to claim 3(b), wherein the acid acceptor is sodium carbonate or bicarbonate, and the reduction is carried out by catalytic hydrogenation.

6. A process according to claim 3(a), characterised in that 2-[2-hydroxy-2-(4-methanesulphonamidophenyl)ethyl]-5-(4-pyridyl)-2,5-diazabicyclo[2.2.1]heptane is prepared by reacting 2-[2-hydroxy-2-(4-methanesulphonamidophenyl)ethyl]-2,5-diazabicyclo[2.2.1]heptane with 4-chloropyridine hydrochloride in the presence of an acid acceptor.

Claims for the following Contracting State: GR

1. A process for preparing a compound of the formula:-

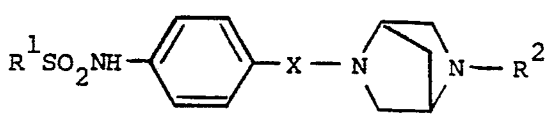

--- (I)

or a pharmaceutically acceptable salt thereof, wherein $R^1$ is $C_1$-$C_4$ alkyl; X is $-(CH_2)_2-$, $-CH(OH)CH_2-$, $-COCH_2-$ or a direct link; and $R^2$ is 4-pyridyl, comprising the reaction of a compound of the formula:-

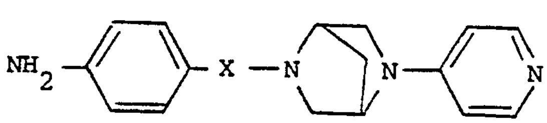

--- (II)

wherein X is as defined for formula (I), with either (a) a $C_1$-$C_4$ alkanesulphonyl chloride or bromide; or (b) a $C_1$-$C_4$ alkanesulphonic anhydride, followed by, optionally, conversion of the product into a pharmaceutically acceptable salt.

2. A process as claimed in claim 1 wherein a compound of the formula (II) is reacted with a $C_1$-$C_4$ alkanesulphonyl chloride in pyridine.

3. A process for preparing a compound of the formula (I) as defined in claim 1, or a pharmaceutically acceptable salt thereof, wherein $R^2$ is 4-pyridyl or 4-amino-2-pyridyl and $R^1$ and X are as defined for formula (I), comprising the reaction of a compound of the formula:

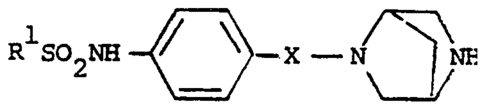

(III)

wherein $R^1$ and X are as defined for formula (I), with, as appropriate, either
(a) an acid addition salt of a compound of the formula:-

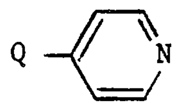 ,

wherein Q is a suitable leaving group, in the presence of a suitable acid acceptor; or
(b) 2-chloro-4-nitropyridine in the presence of a suitable acid acceptor, followed by reduction of the resulting intermediate wherein $R^2$ is 4-nitro-2-pyridyl to give a compound of the formula (I) in which $R^2$ is 4-amino-2-pyridyl;
said process (a) or (b) being followed by, optionally, conversion of the product into a pharmaceutically acceptable salt.

4. A process according to claim 3(a), wherein Q is Cl, the acid addition salt is the hydrochloride, and the acid acceptor triethylamine.

5. A process according to claim 3(b), wherein the acid acceptor is sodium carbonate or bicarbonate, and the reduction is carried out by catalytic hydrogenation.

6. A process according to claim 3(a), characterised in that 2-[2-hydroxy-2-(4-methanesulphonamidophenyl)ethyl]-5-(4-pyridyl)-2,5-diazabicyclo[2.2.1]heptane is prepared by reacting 2-[2-hydroxy-2-(4-methanesulphonamidophenyl)ethyl]-2,5-diazabicyclo[2.2.1]heptane with 4-chloropyridine hydrochloride in the presence of an acid acceptor.

7. A compound of the formula (II) as defined in claim 1.

8. A compound of the formula (III) as defined in claim 3.